# EUROPÄISCHE PATENTANMELDUNG

(11) **EP 1 914 534 A2**
(43) Veröffentlichungstag der Anmeldung: **23.04.2008**
(21) Anmeldenummer: 07017542.7
(22) Anmeldetag: 07.09.2007
(51) Int. Cl.: G01N 1/12

(54) **Eintauchenlanze zur Analyse von Schmelzen und Flüssigkeiten**

(30) Priorität: 06.10.2006 DE 102006047765
(71) Anmelder: Heraeus Electro-Nite International N.V., 3540 Houthalen (BE)
(72) Erfinder: Sattmann, Ralph, 63739 Aschaffenburg (DE)
(74) Vertreter: Kühn, Hans-Christian

(57) **Zusammenfassung**

Die Erfindung betrifft einen Eintauchsensor zur Analyse von Flüssigkeiten oder von Schmelzen mit einem Eintauchträger, der einen Detektor und eine Strahlungsführungseinheit umfasst sowie mit einer in dem Eintauchträger angeordneten Probenkammer mit einer Einlauföffnung, wobei der Sensor zur Messung der Schmelze innerhalb der Probenkammern ausgebildet ist.

## Beschreibung

Die Erfindung betrifft einen Eintauchsensor zur Analyse von Flüssigkeiten oder Schmelzen mit einem Eintauchträger umfassend eine in dem Eintauchträger angeordnete Probekammer mit einer Einlauföffnung.

### Stand der Technik:

Eintauchsensoren sind bereits in verschiedenen Ausführungsformen bekannt. So beschreibt die WO 03/081287 A2 ein Trägerrohr, welches in eine Aluminiumschmelze eingetaucht wird. Innerhalb des Trägerrohres ist ein Linsensystem angeordnet. Am oberen Ende des Rohres befindet sich ein Lichtleiter, der mit einem Spektrographen einerseits und einem Laser andererseits über ein optisches System verbunden ist. Die von der Schmelze ausgehende Strahlung wird über den Lichtleiter in den Spektrographen geleitet, dort wird die Strahlung analysiert, um daraus Analyseergebnisse zur Zusammensetzung der Aluminiumschmelze abzuleiten.

Die DE 103 59 447 A1 beschreibt ebenfalls einen Eintauchsensor zur Analyse von Metallschmelzen mit einem Eintauchträger, mit einem Detektor sowie mit einer Strahlungsführungseinrichtung zur Aufnahme und Weiterleitung von Strahlung und mit einer an oder in dem Eintauchträger angeordneten Signalschnittstelle. Dabei ist die Signalschnittstelle mit dem Detektor verbunden.

### Aufgabenstellung:

Aufgabe der vorliegenden Erfindung ist es, die vorhandenen Vorrichtungen zu verbessern, die Handhabung zu vereinfachen und eine genauere Analyse von Schmelzen und/oder Flüssigkeit zu ermöglichen.

Diese Aufgabe wird bereits mit den Merkmalen des unabhängigen Anspruchs gelöst.

Vorteilhafte Weiterbildungen sind den jeweiligen Unteransprüchen zu entnehmen.

Der erfindungsgemäße Eintauchsensor zur Analyse von Flüssigkeiten oder von Schmelzen mit einem Eintauchträger, der eine in dem Eintauchträger angeordnete Probekammer mit einer Einlauföffnung aufweist, sieht vor, dass der Sensor zur Messung der Schmelze in der Probenkammer ausgebildet ist.

Bei den Flüssigkeiten oder Schmelzen handelt es sich bevorzugt um Glas- oder Metallschmelzen, insbesondere um Aluminium- oder Stahlschmelzen.

Der Sensor ist auf einen vorher bestimmten Punkt in der Probenkammer gerichtet. An diesem Punkt erfolgt die Analyse. Die zu analysierende Flüssigkeit oder Schmelze wird diesem Punkt so zugeführt, dass die freie Oberfläche im Messbereich des Sensors liegt.

Bei der Analyse kann eine Anregung der Flüssigkeit oder Schmelze erfolgen. Dabei wird beispielsweise mittels einer Strahlerzeugungseinheit ein Strahl erzeugt und in der Probenkammer auf den vorher bestimmten Punkt gerichtet. Bei dem Strahl kann es sich um einen Laserstrahl handeln, es sind aber durchaus auch andere Strahlformen denkbar. Der Strahl erzeugt Teilchen und/oder Strahlung an der definierten Messstelle, welche emittiert werden und zu einer Aufnahmeeinrichtung geführt werden. Bei der Aufnahmeeinrichtung handelt es sich insbesondere um einen Detektor, einen Strahlungswandler, ein Spektrometer, ein Röntgen- oder Massenspektrometer. Die Messung kann auf optische Weise erfolgen beispielsweise als Temperaturmessung oder zur Bestimmung der chemischen Zusammensetzung beispielsweise durch LIBS (laser-induced breakdown spectroscopy).

Eine Analyse in einer Probenkammer führt zu besonders genauen Messergebnissen, da im Bereich des Messpunktes eine für die Analyse geeignete Gasatmosphäre hergestellt werden kann, ohne dabei die Lage des Messpunktes zu verändern.

Auch wird bei einer Analyse innerhalb der Probenkammer vermieden, dass Wellen und Bewegungen der Schmelze das Messergebnis verfälschen. Die Analyse an der fließenden Flüssigkeit oder Schmelze verringert Einflüsse durch die Messung selbst z.B. eine An- oder Abreicherung einzelner Elemente durch die Anregung und ergibt eine bessere Signifikanz der Analyse als wenn immer das gleiche Probenvolumen gemessen wird, oder sich die Zusammensetzung der Schmelze durch die Messung verändert.

Unter Analyse wird hier die Messung, also das Ermitteln eines Wertes durch quantitativen Vergleich der Messgröße mit einer Skala, insbesondere von chemischen oder physikalischen Werten verstanden.

Ein erfindungsgemäßer Eintauchsensor ermöglicht Analysen und Messungen der Schmelze an verschiedenen Stellen in der Schmelze, da eine Lageänderung des Sensors einfach durchführbar ist.

Vorteilhafterweise ist der Sensor auf einen vordefinierten Messpunkt innerhalb der Probenkammer ausgerichtet, an dem die frisch einlaufende Schmelze oder Flüssigkeit vorbeigeführt wird. Dies gewährleistet einen definierten Abstand zwischen dem Sensor und der Schmelzoberfläche. Dies führt zu besonders genauen und vergleichbaren Ergebnissen.

Eine vorteilhafte Ausführungsform der Erfindung sieht vor, dass der vordefinierte Messpunkt an einer Eintrittsöffnung der Schmelze in die Probekammer liegt. Es hat sich gezeigt, dass dadurch eine genaue Messung der Zusammensetzung der Schmelze möglich ist, da ständig frische Schmelze zugeführt wird und nur eine sehr geringe Abkühlung der Schmelze erfolgt ist. Die Signifikanz wird verbessert und gleichzeitig eine Veränderung der Zusammensetzung der Schmelze durch die Analyse verhindert, da ja immer frische Schmelze zugeführt wird.

Vorteilhafterweise ist der Messpunkt an oder auf einem Analyseteller angeordnet. Der Analyseteller ermöglicht ebenfalls einen definierten Abstand zwischen Sensor und Schmelze. Auch trägt der Analyseteller dazu bei, dass die Fliesgeschwindigkeit der Schmelze verringert und die Oberfläche der Schmelze vergrößert wird, und so eine genauere Analyse erfolgen kann.

Vorteilhafterweise ist die Eintrittsöffnung ein Einlaufrohr. Es hat sich gezeigt, dass durch ein Einlaufrohr sichergestellt werden kann, dass während der überwiegenden Dauer der Analyse nur reine Schmelze dem Messpunkt zugeführt wird. Es wird verhindert, dass Schlacke und sonstige Ablagerungen das Analyseergebnis verfälschen. Das Einlaufrohr kann auch derart geformt sein, dass die Einströmgeschwindigkeit der Schmelze verringert wird. So kann die Einströmgeschwindigkeit beispielsweise durch eine Biegung oder Verengung des Einlaufrohres kontrolliert werden.

Die Schmelze wird unterhalb der Messstelle in der Probenkammer gesammelt.

Vorteilhafterweise weist der Eintauchsensor einen Schmelzpegeldetektor auf. Dieser Schmelzpegeldetektor misst den Pegelstand der Schmelze in der Probenkammer und ermöglicht, dass der Sensor aus der Schmelze gezogen werden kann, wenn die Probenkammer bis zu einem definierten Pegelstand mit Schmelze gefüllt ist. Dadurch kann eine Schädigung des Sensors und der in dem Sensor enthaltenden Optik vermieden werden. Bei einem derartigen Schmelzpegeldetektor kann es sich um Kontaktfühler, Ultraschallsensoren, optische Sensoren oder Ähnliches handeln. Es sind hier sämtliche weitere Vorrichtungen denkbar, die eine Messung des Pegelstandes ermöglichen.

Es ist durchaus denkbar, dass der Pegeldetektor mit einer Einrichtung verbunden ist, die bei einem definierten Pegelstand ein automatisches Herausziehen des Sensors aus der Schmelze ermöglicht.

Dabei ist es ferner von Vorteil, wenn die Optik oder andere empfindliche Teile mit einem Schutzfenster vor Spritzern oder Dämpfen der Schmelze geschützt werden.

Vorteilhaft ist es, wenn der Eintauchträger als Rohr ausgebildet ist. Dadurch lassen sich die einzelnen Teile gut im Eintauchsensor anordnen und sind beim Transport geschützt.

Vorteilhafterweise weist der Detektor eine Einrichtung zur Aufnahme von Strahlung und zur Umwandlung in elektrische Signale auf, insbesondere ist der Detektor zur Aufnahme und Umwandlung von sichtbarem Licht, Ultraviolettstrahlung, Infrarotstrahlung, Röntgenstrahlung und/oder Mikrowellenstrahlung in elektrische Signale eingereichtet. Damit lassen sich alle Arten von optischen oder anderen Strahlungen aufnehmen und zur Analyse der Schmelze nutzbar machen.

Von Vorteil ist es, wenn an oder in dem Eintauchträger ein optisches Spektrometer, ein Röntgenspektrometer und/oder ein Massenspektrometer angeordnet ist.

Eine vorteilhafte Ausgestaltungsform der Erfindung sieht vor, dass der Eintauchsensor modular aufgebaut ist, bevorzugt zweigeteilt. Dabei ist ein Teil, vorteilhafterweise der obere Teil, ein wiederverwendbarer Teil, welcher die Einrichtungen zum Analysieren enthält. Der untere Teil ist für die einmalige Verwendung ausgerichtet und enthält die Probenkammer. Der obere, wiederverwendbare Teil kann während des Analyseverfahrens vollständig oberhalb der Schmelze verbleiben.

Um den Eintauchsensor vor der Hitze zu schützen, ist es vorteilhaft, wenn der Eintauchsensor wassergekühlt ist. Dadurch werden längere Analysezeiten ermöglicht was zu genaueren Analyseergebnissen führt.

Vorteilhaft ist es, wenn der Eintauchsensor eine Schutzkappe aufweist, welche sich an der Eintrittsöffnung befindet und die erst nach einer gewissen Zeit nach dem Eintauchen wegschmilzt. Dadurch kann gewährleistet werden, das nur saubere Schmelze, also keine Schlacke an den Messpunkt gelangt und ein genaues Messen und Analysieren ermöglicht wird.

Die Erfindung wird nachfolgend anhand bevorzugter Ausführungsformen und unter Bezugnahme der beigefügten Figuren näher erläutert.

Dabei zeigt in schematischer Darstellung:
- Figur 1: Querschnitt durch einen erfindungsgemäßen Eintauchsensor
- Figur 2: Ansicht der Probenkammer mit Messpunkt
- Figur 3: Darstellungen des Messtellers
- Figur 4: eine weitere Ansicht der Probenkammer mit Messpunkt
- Figur 5: Vorrichtung zum Messen und Analysieren von Schmelzen
- Figur 6: eine weitere Ansicht der Probenkammer mit Messpunkt.

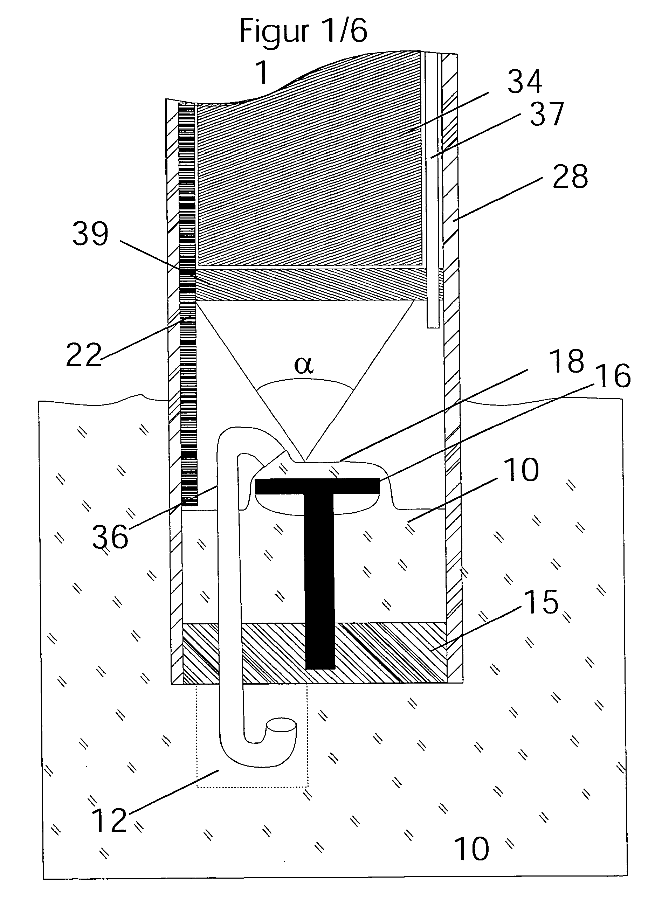

Figur 1 zeigt einen erfindungsgemäßen Eintauchsensor 1 im Querschnitt. Der Eintauchsensor 1 ist von einem Schutzrohr 28 umgeben. Am unteren Ende des Eintauchsensors 1 befindet sich die Probenkammer 17. Innerhalb der Probenkammer 17 ist ein Schmelzpegeldetektor 22 , ein Probenteller 16 und eine Einlassöffnung 14, die in diesem Fall durch ein Eintrittsrohr 36 gebildet wird. Das der Schmelze 10 zugewandte Ende des Eintrittsrohres 36 ist mit einer Schutzkappe 12 versehen, die nach dem Eintauchen des Eintauchsensors 1 in die Schmelze 10 wegschmilzt und somit sicherstellt, dass nur saubere Schmelze 10 an den Messpunkt 18 gelangt. Beim Eintauchen des Eintauchsensors 1 in die Schmelze 10 löst sich die Schutzkappe 12 auf und die Schmelze 10 gelangt durch die Einlassöffnung 14 in die Probenkammer 17. Beim Eintritt der Schmelze 10 in die Probenkammer 17 wird die Schmelze 10 an einen definiertem Messpunkt 18 analysiert. Der Messpunkt 18 ist in der Figur 1 auf einem Probenteller 16 angeordnet. Dabei kann der Probenteller 16 an jeder beliebigen Stelle innerhalb der Probenkammer 17 angeordnet sein. Am Boden der Probenkammer 17 wird die Schmelze10, die durch die Einlassöffnung 14 in die Probenkammer 17 gelangt, aufgefangen. Diese kann bei Bedarf beim Herausnehmen des Eintauchsensors 1 mit entnommen und weiteren Analysen zugeführt werden. Im oberen Teil des Eintauchsensors 1 befindet sich die Optik 34., sowie eine Gaszuführung 37 zum Zuführen von Gas um einen bestimmten Druck innerhalb der Probenkammer 17 zu ermöglichen.

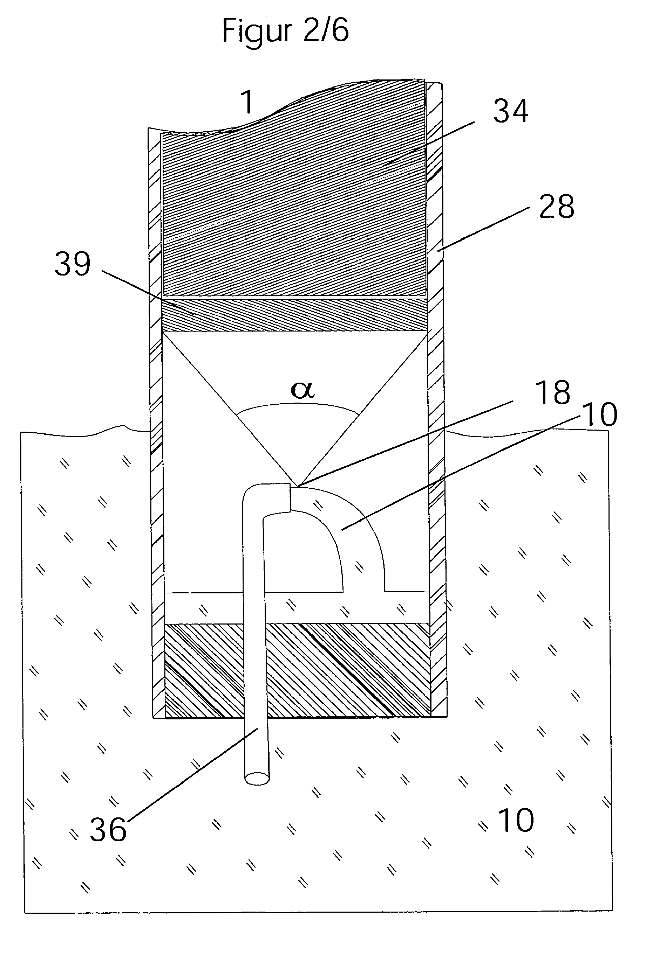

Figur 2 zeigt die Ansicht der Probenkammer 17 mit Messpunkt 18. Dabei ist der Messpunkt 18 nicht auf einem Probenteller 16 angeordnet, sondern die Messung der Schmelze 10 erfolgt bei deren Eintritt aus der Einlassöffnung 14 in die Probenkammer 17. Der Eintritt der Schmelze 10 erfolgt mittels eines Eintrittrohres 36.

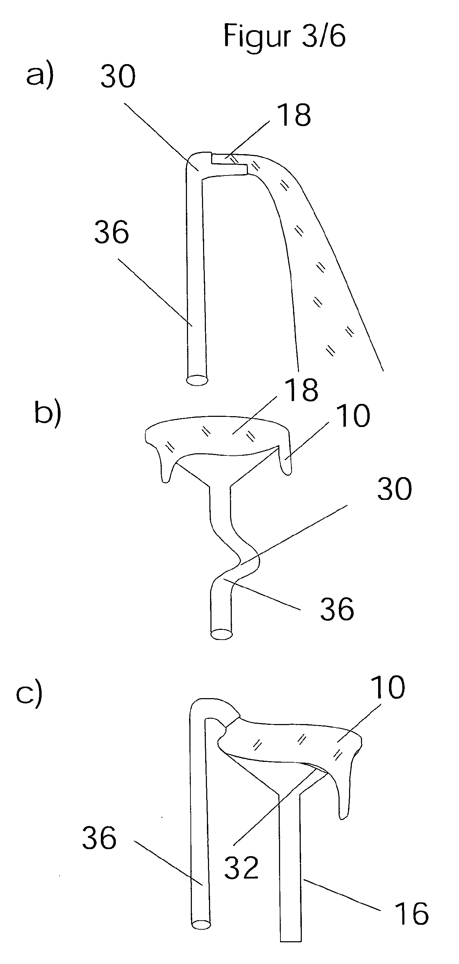

Figur 3 zeigt verschiedene Ausgestaltungsformen des Eintrittrohres 36. In Figur 3a ist das Eintrittsrohr 36 derart ausgestaltet, dass die Schmelze 10 einen Bogen 30 durchfließen muss. Dabei weist das Eintrittsrohr 36 an seinem Ende, welches sich in der Probenkammer 17 befindet, einen Bereich auf, der nach oben hin abgefräst ist. An diesem Bereich kann die Schmelze 10 besonders gut analysiert werden, weshalb dieser Bereich eine Art Probenteller 16 bildet.

Figur 3b zeigt eine weitere Ausgestaltungsform des Eintrittrohres 36. Dabei weist das Eintrittsrohr 36 einen Bogen 30 auf, der ein zu schnelles Einströmen der Schmelze 10 in die Probenkammer 17 verhindern soll. Der obere Bereich des Eintrittrohres 36 ist als Probenteller 16 ausgebildet. Die Schmelze 10 fließt über den Rand des Probentellers 16 und gelangt so in die Probenkammer 17. Die Messung der Schmelze 10 kann entweder auf dem Probenteller 16 oder beim Überlaufen der Schmelze 10 über den Rand des Probetellers 16 erfolgen.

In Figur 3c ist eine weitere Möglichkeit dargestellt, wie die Schmelze 10 analysiert werden kann. Die Schmelze 10 gelangt über ein Eintrittsrohr 36 in die Probenkammer 17 und fließt dort auf einen Probenteller 16. Der Probenteller 16 weißt einen Überlauf 32 auf, an dem das kontrollierte Abfließen der Schmelze 10 erfolgt.

Der Probenteller 16 kann je nach Bedarf eben, außen hochgewölbt oder in der Mitte hochgewölbt ausgelegt sein, oder eine komplizierte Form aufweisen und besondere Merkmale wie beispielsweise den Überlauf 32 aufweisen. Dabei können Eintrittsrohr 36 und Probenteller 16 separate Bauteile sein, oder monolithisch in die Probenkammer 17 integriert sein. Um Verunreinigungen der Schmelze 10 vor der Analyse zu minimieren, kann reines Quarzglas als Eintrittsrohr 36 eingesetzt werden. Es sind hier aber auch Zement, Keramik oder ähnliche Materialien als Eintrittsrohr 36 und Probenteller 16 denkbar.

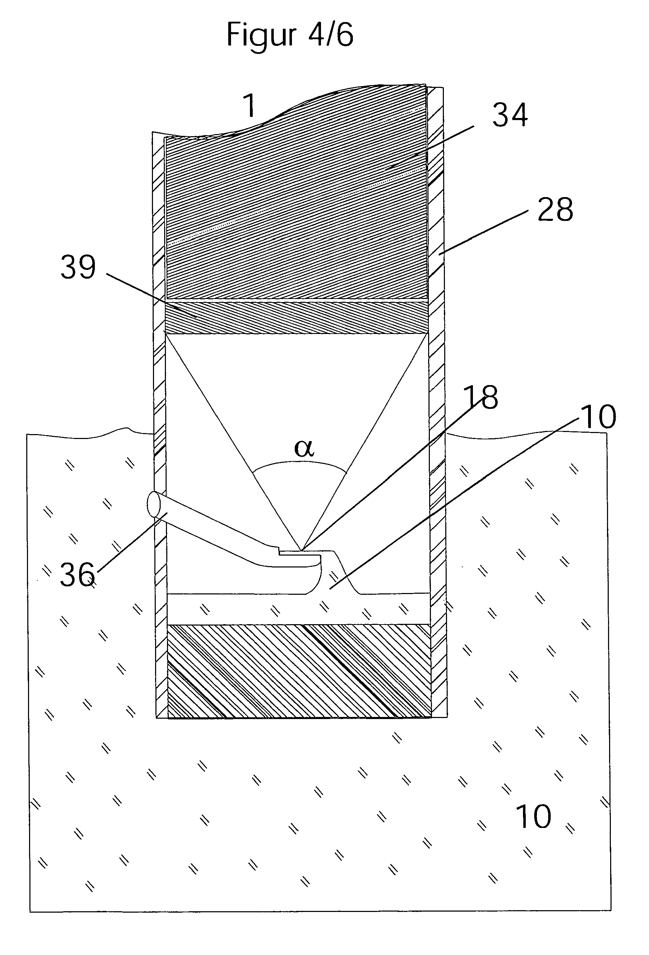

Figur 4 zeigt eine weitere Ansicht der Probenkammer 17 mit Messpunkt 18. Dabei wird über ein Eintrittsrohr 36, welches sich an der Seite des Eintauchsensors 1 befindet, die Schmelze 10 in die Probenkammer 17 eingebracht. Die Messung erfolgt hierbei beim Auslaufen der Schmelze 10 aus dem Eintrittsrohr 36. Es ist hierbei denkbar, dass die Schmelze 10 auch auf einen Probeteller fließen kann.

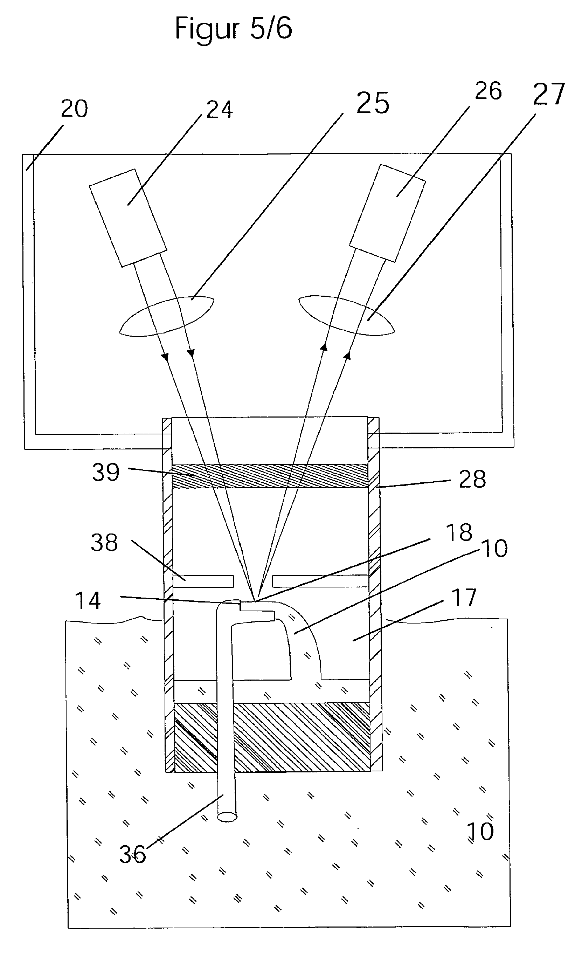

Figur 5 zeigt eine Vorrichtung zum Messen und Analysieren von Schmelzen. Der hier abgebildete Eintauchsensor 1 weist einen oberen, wiederverwendbaren Teil 20 und einen unteren Teil auf. Innerhalb des Eintauchsensor 1 befindet sich ein Laser 24 und ein Spektrometer 26. Der Eintauchsensor 1 weist ein Gehäuse 28 auf, welches wassergekühlt sein kann. Im unteren Teil des Eintauchsensor 1 befindet sich die Einlassöffnung 14 mit einem Eintrittsrohr 36 durch das die Schmelze 10 beim Eintauchen des Eintauchsensor 1 in die Schmelze 10 in die Probenkammer 17 gelangt. Dabei dient eine Quarzglasscheibe 38 als Schutzfenster 39 zum Schutz des Lasers 28 vor Dämpfen oder Wärmestrahlung der Schmelze 10.

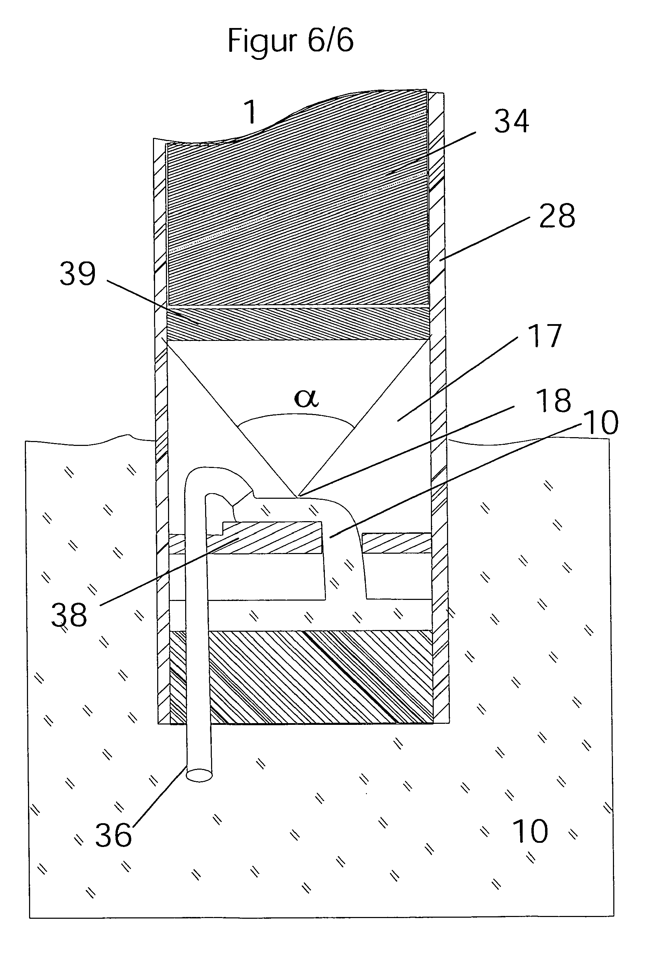

In Figur 6 ist eine weitere erfindungsgemäße Ausgestaltungsform des Eintauchsensors 1 dargestellt. Der Eintauchsensor 1 weist hier in der Probenkammer 17 eine Platte 38 auf, welche unter anderem die Funktion des Probentellers 16 übernimmt.

### Bezugszeichenliste:

- 1: Eintauchsensor
- 10: Schmelze
- 12: Schutzkappe
- 14: Einlassöffnung
- 16: Probenteller
- 17: Probenkammer
- 18: Messpunkt
- 20: Schutzrohr
- 22: Schmelzpegeldetektor
- 24: Laser
- 26: Sprektrometer
- 28: Gehäuse
- 30: Bogen
- 32: Überlauf
- 34: Optik
- 35: Eintrittsrohr
- 37: Gassupply
- 38: Platte
- 39: Schutzfenster

## Patentansprüche

1. Eintauchsensor zur Analyse von Flüssigkeiten oder Schmelzen umfassend einen Eintauchträger mit einer in dem Eintauchträger angeordneten Probenkammer mit einer Einlauföffnung, **dadurch gekennzeichnet, dass** der Sensor zur Messung der Flüssigkeit oder Schmelze in der Probenkammer ausgebildet ist.

2. Eintauchsensor nach Anspruch 1, **dadurch gekennzeichnet, dass** der Sensor auf einen vordefinierten Messpunkt innerhalb der Probenkammer ausgerichtet ist.

3. Eintauchsensor nach einem oder mehreren der vorhergegangenen Ansprüche, **dadurch gekennzeichnet, dass** der vordefinierte Messpunkt an einer Eintrittsöffnung der Schmelze in die Probenkammer liegt.

4. Eintauchsensor nach einem oder mehreren der vorhergegangenen Ansprüche, **dadurch gekennzeichnet, dass** die Analyse an der einlaufenden Schmelze erfolgt.

5. Eintauchsensor nach einem oder mehreren der vorhergegangenen Ansprüche, **dadurch gekennzeichnet, dass** der Messpunkt an einem Analyseteller angeordnet ist.

6. Eintauchsensor nach einem oder mehreren der vorhergegangenen Ansprüche, **dadurch gekennzeichnet, dass** die Eintrittsöffnung ein Einlaufrohr ist.

7. Eintauchsensor nach einem oder mehreren der vorhergegangenen Ansprüche, **dadurch gekennzeichnet, dass** der Eintauchsensor einen Schmelzpegeldetektor aufweist.

8. Eintauchsensor nach einem oder mehreren der vorhergegangenen Ansprüche, **dadurch gekennzeichnet, dass** der Eintauchträger als Rohr ausgebildet ist.

9. Eintauchsensor nach einem oder mehreren der vorhergegangenen Ansprüche, **dadurch gekennzeichnet, dass** der Sensor zur Messung physikalischer und /oder chemischer Parameter ausgebildet ist.

10. Eintauchsensor nach einem oder mehreren der vorhergegangenen Ansprüche, **dadurch gekennzeichnet, dass** an oder in dem Eintauchträger ein optisches Spektrometer, ein Röntgenspektrometer und/oder ein Massenspektrometer angeordnet ist.

11. Eintauchsensor nach einem oder mehreren der vorhergegangenen Ansprüche, **dadurch gekennzeichnet, dass** der Sensor Komponenten zur Anregung der Flüssigkeit oder Schmelze mit Strahlung aufweist.

12. Eintauchsensor nach einem oder mehreren der vorhergegangenen Ansprüche, **dadurch gekennzeichnet, dass** dieser modular aufgebaut ist.

13. Eintauchsensor nach einem oder mehreren der vorhergegangenen Ansprüche, **dadurch gekennzeichnet, dass** dieser wassergekühlt ist.

14. Eintauchsensor nach einem oder mehreren der vorhergegangenen Ansprüche, **dadurch gekennzeichnet, dass** die Einlassöffnung eine Schutzkappe aufweist.

15. Verfahren zum Analysieren von Schmelzen mittels eines Eintauchsensor nach einem der Ansprüche 1 bis 14.

16. Verwendung eines Eintauchsensors nach einem der Ansprüche 1 bis 14 zum Analysieren von Schmelzen, insbesondere von Metallschmelzen.
